# EUROPEAN PATENT APPLICATION

(11) **EP 3 549 579 A1**
(43) Date of publication of application: **09.10.2019**
(21) Application number: 18305393.3
(22) Date of filing: 03.04.2018
(51) Int. Cl.: A61K 9/68, A61K 31/167, A61K 31/445, A61K 33/08, A61K 36/185, A61K 36/53

(54) **ORAL GUM FORMULATION AND FABRICATION PROCESS THEREOF**

(71) Applicant: Sanofi Winthrop Industrie, 92160 Antony (FR)
(72) Inventor: The designation of the inventor has not yet been filed
(74) Representative: Santarelli

(57) **Abstract**

The present invention relates to a chewable oral gum (OG) formulation comprising at least one pharmaceutically active ingredient. The formulation comprises gelatin, glycerol, sorbitol and water, present in a specific ratio. The present invention also relates to a process particularly well-suited to prepare said chewable oral gum formulation.

## Description

### TECHNICAL AREA OF THE INVENTION

The present invention relates to a chewable oral gum (OG) formulation comprising at least one pharmaceutically active ingredient. The formulation comprises gelatin, glycerol, sorbitol and water, present in a specific ratio. The present invention also relates to a process particularly well-suited to prepare said chewable oral gum formulation.

### BACKGROUND OF THE INVENTION

Physical characteristics, like solubility or compressibility, of drug substances, determine, to a large extent, their formulation form and size. To administer an effective dose, the dosage form, for example, tablet or capsule, has to be of a certain minimum size. Thus, some patients, especially elderly patients and children, may have difficulty in swallowing relatively large tablets or capsules.

Furthermore, administration of a tablet usually requires the ingestion of a liquid to facilitate swallowing. Often, patients do not always have a liquid at hand, and thus may defer taking the drug substance until they can have a glass of water beside them, or simply not take it at all.

Similarly, children may be simply unable to swallow the solid dosage form, even with water, and may refuse to take it. Breaking tablets or capsules in two to reduce the size almost certainly results in imprecise dosing. Putting broken tablets in the mouth often have a bitter taste or chalky sensation (coming from the composition inside the tablet). This all adds to the already complicated task of administering medicaments to children, to the elderly and to those with swallowing difficulties.

These issues can result in poor compliance or non-compliance with a treatment.

Thus, there is still a need for new oral pharmaceutical formulations that are convenient for most patients, especially the elderly and pediatric population, and for those patients having swallowing difficulty or other difficulties related to ingestion of tablets. In particular, such a formulation should be easy to swallow and preferably be ingested without liquid.

The present invention provides a pharmaceutical oral gum formulation, especially suitable for the above mentioned populations, as an alternative to solid dosage forms.

To manufacture an oral gum formulation, the ingredients are usually heated in a solution with a gelling agent. The molten solution is then poured into a mold and the resulting gum units are left to dry. For example, in known processes for producing candy, the liquid gum (usually at high temperature, above 90°C) is usually poured into reusable wooden, ceramic or hard plastic molds, and then the hardened gum units are emptied onto a conveyor belt and transported for packaging. This type of process is not suitable for pharmaceutical gum manufacture, because it is not compliant with quality and regulation constraints applied to pharmaceutical processes, for example, because of the risk of cross-contamination of ingredients.

Furthermore, the high temperatures used in the candy making processes damage heat sensitive pharmaceutically active ingredients.

In patent application US20160067180A1, from SANTA-CRUZ PHARMACEUTICALS, an oral gum medication is provided containing a mixture of gelatin and pectin. The heating temperatures used are above 100°C, and, therefore, the process is not suitable for heat sensitive active ingredients.

Similarly, in the patent application WO2014124981A1, from SANOFI related to chewable composition for oral administration comprising high (methyl)pectin, the relatively high processing temperatures (above 90°C) preclude the inclusion of heat sensitive active ingredients.

There is a need to provide a pharmaceutical formulation suitable for dosing pharmaceutical ingredients including those that are heat sensitive, and in a form that is suitable for administration without ingestion of liquid. There is a need to provide a pharmaceutical dosage suitable for administration to children, elderly and those patients having difficulty swallowing tablet or liquid dosage forms. There is a need to provide a process suitable for the manufacture of such pharmaceutical formulations, including those formulations comprising heat sensitive active ingredients.

### SUMMARY OF THE INVENTION

According to one aspect, the invention concerns a chewable oral gum pharmaceutical formulation comprising water, sorbitol, gelatin and glycerol and at least one pharmaceutically active agent characterized in that with respect to water:
- the ratio of gelatin is about 0.20 - 0.80:1 by weight,
- the ratio of glycerol is about 0.65 - 3.50:1 by weight,
- the ratio of sorbitol is about 0.15 - 1.45:1 by weight.

According to one embodiment of the invention:
- the ratio of gelatin to water is from about 0.28 - 0.70:1 by weight,
   - the ratio of glycerol to water is from about 0.70 - 3.00:1 by weight,
   - the ratio of sorbitol to water is from about 0.20 - 1.40:1 by weight.

According to one embodiment of the invention, the amount of gelatin is about 4 - 12% w/w, the amount of water is about 15 - 30% w/w, the amount of sorbitol is about 6 - 30% w/w, and the amount of glycerol is from about 30 to about 60 % w/w.

According to one embodiment of the invention, the water activity (Aw) of said formulation is less than 0.75, preferably less than 0.70 and more preferably less than 0.61.

According to one embodiment of the invention, the at least one pharmaceutically active ingredient is chosen from fexofenadine, the combination of magnesium hydroxide and aluminum hydroxide, thyme extract, primula extract or a combination of thyme extract and primula extract, Diphenhydramine, Chlorpheniramine, Loratidine, Cetirizine, Pseudoephedrine, Guaifenesin, Dextromethorphan, Naproxen, Aspirin, Acetaminophen, Ibuprofen, Fluriprofen, Ketoprofen, Drotaverine, Codeine, magnesium salts, for example, citrate, Silibinin, Ambroxol, Hyoscine Butyl Bromide, Bromhexine, Dextromethorphan, picosulphate, or pharmaceutically acceptable salts thereof, or one or more probiotic strains, vitamin A, K, D, E, C, B1, B2, B3, B5, B6, B7, B9, or B12, or multivitamin compositions, estrogen and estrogen derivatives, unsaturated fatty acids, flavonoids, phytosterol, and combinations thereof.

According to one embodiment of the invention, the at least one pharmaceutically active ingredient is chosen from fexofenadine, the combination of magnesium hydroxide and aluminum hydroxide, thyme extract, primula extract or a combination of thyme extract and primula extract and Acetaminophen, or pharmaceutically acceptable salts thereof.

According to one embodiment of the invention, the at least one pharmaceutically active ingredient is fexofenadine, or pharmaceutically acceptable salts thereof, and with respect to water:
- the ratio of gelatin is from about 0.3 - 0.4:1 by weight,
- the ratio of glycerol is from about 0.7 - 1.3:1 by weight,
- the ratio of sorbitol is from about 0.4:1 - 1.4:1 by weight.

According to one embodiment of the invention, the at least one pharmaceutically active ingredient is a combination of magnesium and aluminum hydroxides and with respect to water:
- the ratio of gelatin is from about 0.2 - 0.35:1 by weight,
- the ratio of glycerol is from about 1.2 - 1.5:1 by weight,
- the ratio of sorbitol is from about 0.1:1 - 0.4:1 by weight.

According to one embodiment of the invention, the at least one pharmaceutically active ingredient is a combination of primula and thyme plant extracts and with respect to water:
- the ratio of gelatin is from about 0.4 - 0.7:1 by weight,
- the ratio of glycerol is from about 1.3 - 2.9:1 by weight,
- the ratio of sorbitol is from about 0.3 - 0.6:1 by weight.

According to one embodiment of the invention, the at least one pharmaceutically active ingredient is Acetaminophen, or pharmaceutically acceptable salts thereof, and with respect to water:
- the ratio of gelatin is from about 0.1:1 to about 0.4:1 by weight,
- the ratio of glycerol is from about 1.2:1 to about 1.6:1 by weight,
- the ratio of sorbitol is from about 0.15:1 to about 0.3:1 by weight.

Generally, the pharmaceutical oral gum formulation is stable and does not require preservatives. The formulation has acceptable organoleptic properties.

The pharmaceutical oral gum formulation may be produced in a pharmaceutical manufacturing process.

Therefore, according to another aspect, the invention also concerns a process for preparing the above mentioned chewable oral gum pharmaceutical formulations comprising the steps of:
(i) adding the glycerol,
(ii) adding the sorbitol,
(iii) adding the water,
(iv) adding the gelatin,
(v) heating the mixture to a temperature of about 45°C to 75°C,
(vi) adding the at least one pharmaceutically active ingredient,
(vii) optionally, adding the sugar substitute(s) and/or the coloring agent(s) and/or the flavoring agent(s), if present,
(viii) removing air bubbles from the resulting liquid formulation,
(ix) pouring the liquid formulation into (optionally newly) thermoformed blisters using an automated filler, wherein the amount of liquid poured into the blisters is controlled by measuring the level of the liquid formulation in blister and then, if the measured liquid level in that blister differs from a predefined target liquid level, adjusting the amount of the liquid formulation poured into the following blister so that the predefined liquid level is reached,
(x) sealing the blister pack containing the formulation with a film/sheet/cover,
(xi) cooling the filled blister pack,
(xii) optionally, cutting the blister pack into units of suitable size for packaging,
wherein, preferably, the pouring, sealing and cooling steps and the blister thermoforming are carried out in one continual production line.

According to one embodiment of the invention, in step (ix), the target liquid is 1.0 mm - 2.50 mm.

According to one embodiment of the invention, steps (i) to (viii) are carried out in one tank.

According to one embodiment of the invention, the at least one pharmaceutically active ingredient to be added in step (iii) and/or the gelatin added in step (i) is previously dissolved or suspended in a separate tank and added to the main tank from said separate tank.

According to one embodiment of the invention, the temperature of formulation at heating step (ii) and/or pouring step (vi) is about 40°C to about 55°C.

According to one embodiment of the invention, the temperature of formulation at heating step (ii) and/or pouring step (vi) is about 60°C to about 75°C.

The OG formulation may be poured directly into a blister where it hardens upon cooling. The OG may be removed easily from the blister without sticking to it.

### DESCRIPTION OF THE FIGURES

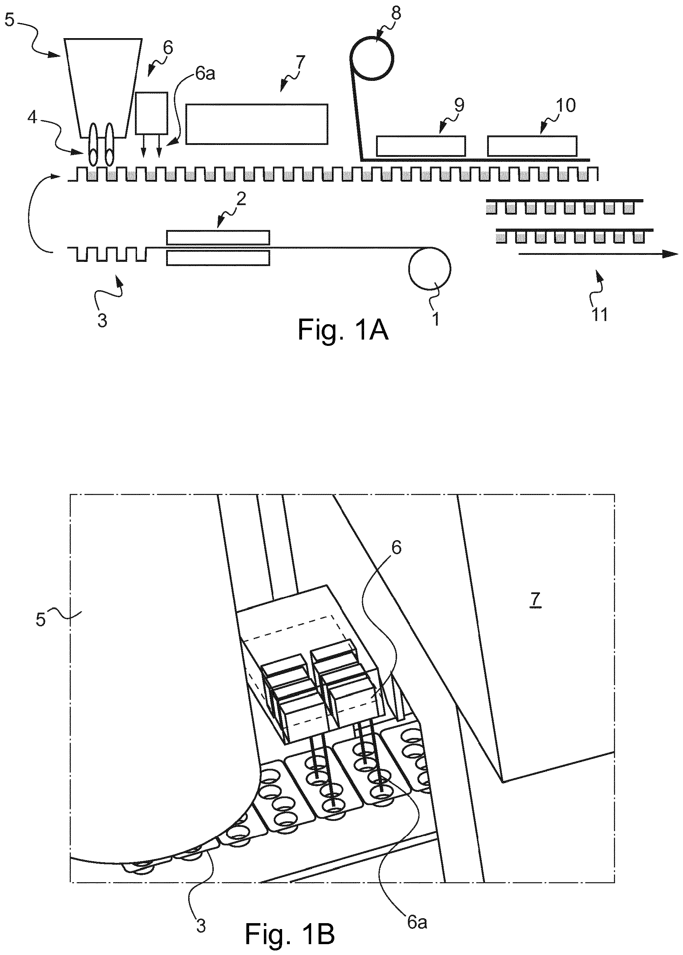

Figure 1A: A schematic diagram of the production process for the manufacture of the chewable oral gum pharmaceutical formulation (1) coil with blister material, (2) heating plate, (3) thermoformed blister, (4) filling nozzles, (5) filling unit /hopper, (6) (laser) measuring unit, (6a) laser beam, (7) cooling unit, (8) sealing film coil, (9) sealing plate, (10) cutting tool.
Figure 1B: Detail of the blisters passing under the filling unit /hopper (5), the liquid level being measured by the measuring unit (6), in particular, with a laser beam (6a).

### DETAILED DESCRIPTION

The formulation of the present invention is in the form of a chewable oral gum that may be easily administered. It has acceptable organoleptic properties and is stable in the absence of preservatives. The inventors have been able to achieve these properties by combining the active ingredient with excipients that are present in a specific ratio.

Thus, sorbitol, gelatin and glycerol are present in the following ratios with respect to water;
- the ratio of gelatin is about 0.20 - 0.80:1 by weight, preferably about 0.28 - 0.70:1 by weight
- the ratio of glycerol is about 0.65 - 3.50:1 by weight, preferably about 0.70 - 3.00:1 by weight
- the ratio of sorbitol is about 0.15 - 1.45:1 by weight, preferably, about 0.20 - 1.40:1 by weight.

It should be noted that the above ratios are calculated with respect to the amount of the pure ingredient present. Therefore, if the sorbitol is added to the formulation as a liquid, for example, as a 70% w/v solution, then the amount of solid sorbitol used to make the solution is used to calculate the ratio with water. Similarly, the 30% water that would be present in such a 70% sorbitol solution is taken into account in the calculation of the total amount of water in the formulation.

While respecting the above ratios of the four components, water, gelatin, glycerol and sorbitol, the chewable OG pharmaceutical formulation generally comprises:
- about 4 - 12% w/w gelatin,
- about 15 - 30% w/w water,
- about 6 - 30% w/w sorbitol,
- about 30 - 60 % w/w glycerol.

The combination of these components in these specific ratios gives the OGs the desired physical and organoleptic properties. The formulations are stable, chewable, palatable and do not stick to their blister packaging when demolded. Furthermore, they may be manufactured in a pharmaceutical industrial process.

The ability of the formulation to form a gum, which contributes to the acceptable organoleptic properties of the composition, largely depends on the nature and quantity of gelling agent, as well as its ratio to the other components in the formulation.

The inventors have found that, generally, if the total amount of gelatin is less than 4% w/w, oral gum may be too fragile and not hold together, whereas if the total amount of gelatin is more than 12% w/w, oral gum structure may be too hard to be chewed comfortably.

The inventors have also found that, generally, if the total amount of water is less than about 15% w/w, excipients and/or active pharmaceutical ingredients may be not totally dispersed; furthermore, if the total amount of water is less than 15% w/w, gelatin may be not fully hydrated.

According to an embodiment of the invention, the ratio of gelatin is 0.20 - 0.80:1 by weight with respect to water. In a preferred embodiment, the ratio of gelatin is 0.28 - 0.70:1 by weight with respect to water.

The inventors have found that the formulations of the invention may, preferably, contain about 4 - 30 % w/w sorbitol. If the total amount of sorbitol is less than 4 % w/w, the oral gum may have a poor structure resulting in an unacceptable consistency for the pharmaceutical formulation. If the total amount of sorbitol is more than 30% w/w, the oral gum may stick to the blister packaging when being removed from it.

Generally, the ratio of sorbitol is about 0.15 - 1.45:1 by weight with respect to water. In a preferred embodiment, the ratio of sorbitol to water is about 0.20- 1.40:1 by weight with respect to water.

Generally, if the total amount of glycerol is less than 30% w/w, oral gum may have a poor structure, resulting in an unacceptable consistency for the formulation. Furthermore, if the total amount of glycerol is less than 30% w/w, other excipients and active pharmaceutical ingredients may be not dissolved properly, as glycerol has a solubilizing function in the formulation. However, if the total amount of glycerol is greater than 60 % w/w, the oral gum may stick to the blister packaging when being removed from it.

Generally, the ratio of glycerol is about 0.65 - 3.50:1 by weight with respect to water. In a preferred embodiment, the ratio of glycerol is from about 0.70 - 3.00:1 by weight with respect to water.

It should be noted that throughout the present application, ranges are intended limits inclusive.

### Active substance:

By "active substance", or "drug substance" or "active ingredient", or "active pharmaceutical ingredient", it is meant a drug substance, optionally with at least one pharmaceutical excipient.

The pharmaceutical formulation of the invention includes one or more active pharmaceutical ingredients that are generally available as over-the-counter medications.

The drug substance is more particularly a molecule or mixture of molecules (which may be present in a neutral form, as a free base, free acid or in a salt form), or mixtures of molecules, intended to treat or prevent at least one disease and/or cure or prevent at least one symptom. This drug substance is introduced into the formulation in an amount corresponding to the usual dosage amount for that particular drug substance.

As an example, the drug substance can be selected among the group consisting of:
- Antacids, antalgic agents, analgesic agents, antipyretic agents, molecules able to treat cough and cold (in particular antitussive agents, decongestant agents and/or expectorants), molecules to treat allergies (antihistaminic agent), antispasmodic agents, antidiarrheal agents, anti-inflammatory agents (such as non-steroidal anti-inflammatory drugs (NSAID)), antispasmodic agents, antimigraine agents, muscle relaxant agents, diuretic agents, or other drug substances, and combinations thereof.

The drug substance may in the form of a plant extract demonstrating positive health benefits. The health benefits are preferably chosen from the treatment or prevention of cough, cold, nasal or bronchial congestion, allergy, diarrhea, inflammation, migraine, menstrual cramps, menopausal symptoms. The drug substance in the form of a plant extract may also be a muscle relaxant, antispasmodic agent, diuretic agent, a decongestant agent or expectorant, antitussive agent or antihistaminic agent.

The plant extract may be in any suitable form for incorporation as a drug substance in the OG formulation. It may be in liquid or powder form.

Preferably, said drug substance is selected among the group consisting of:
- Diphenhydramine, Chlorpheniramine, Loratidine, Cetirizine, Pseudoephedrine, Guaifenesin, Dextromethorphan, Naproxen, Aspirin, Acetaminophen, Ibuprofen, Fluriprofen, Ketoprofen, Drotaverine, Codeine, the combination of magnesium hydroxide and aluminum hydroxide, magnesium salts, for example, citrate, Fexofenadine, or pharmaceutically acceptable salts thereof, thyme extract, primula extract or a combination of thyme extract and primula extract and appropriate combinations thereof,
- Silibinin, Ambroxol, Hyoscine Butyl Bromide, Bromhexine, Dextromethorphan, picosulphate, or pharmaceutically acceptable salts thereof, and combinations thereof.

The drug substance can also be one or more probiotic strains. A probiotic strain is generally a micro-organism that when administered in adequate amounts, confers a health benefit on the host (WHO definition).

Preferred strains are *Bacillus clausii, Bacillus subtilis, Bacilus Licheniformis, Bacilus cereus, Bacillus pumilus, Bacillus coagulans, Bacillus simplex,* and *Bacillus sonorensis.*

The drug substance can also be selected among the group consisting of molecules having prophylactic or nutritional activities such as:
- Vitamins (A, K, D, E, C, B1, B2, B3, B5, B6, B7, B9, B12) or multivitamin compositions, minerals (such as calcium and/or magnesium salts), estrogen and estrogen derivatives, unsaturated fatty acids, flavonoids, phytosterol, and combinations thereof.

More preferably, said drug substance is selected from the group consisting of:
- Fexofenadine, the combination of magnesium hydroxide and aluminum hydroxide, thyme extract, primula extract or a combination of thyme extract and primula extract and Acetaminophen, or pharmaceutically acceptable salts thereof.

### Pharmaceutical excipient:

The drug substance is present in the formulation with water, sorbitol, gelatin and glycerol.

Optionally, other pharmaceutical excipients may be added in limited amounts, described hereafter.

Preferably, the pharmaceutical excipient is selected among the group consisting of:
- excipients to enhance the solubility of the drug substance. As an example, one may cite cyclodextrin;
- buffers, for example, buffers at pH 6.0 to 6.5, preferably, at pH 6.2, such as a phosphate buffer, which may be sodium dihydrogen phosphate, monosodium dihydrogen orthophosphate and monosodium dihydrogen monophosphate;
- coating agents, for example, enteric-resistant polymers. These may be chosen among polymethacrylates (such as those sold under the trade name Eudragit® L), cellulose esters such as hypromellose acetate succinate (HPMCAS) and/or cellulose acetate phthalate (CAP), and/or polyvinyl derivatives such as polyvinyl acetate phthalate (PVAP), etc.;
- coating agents that mask the taste of the drug substance. As an example, taste-masking excipients can be chosen among hydroxypropyl methylcellulose (HPMC), polyvinylpyrrolidone (PVP), polyethylene glycol (abbreviated as PEG, such as PEG 4000 or PEG 400), polymethacrylate, ethylcellulose (EC), stearic acid, diacylglycerides (such as glyceryl palmitostearate sold under the trade name Precirol® ATO 5), polymethacrylates (such as those sold under the trade name Eudragit® EPO), etc.
- surfactants, for example phospholipids or polysorbates, to further facilitate demoulding and further prevent the oral gum sticking to the blister moulds. We may cite the following examples of suitable surfactants; polysobate 80®, macrogol ricinoleate, macrogol 1500-glycerol triricinoleate, lecithin and glycocholic acids.

According to a preferred embodiment of the invention, when the at least one pharmaceutically active ingredient is fexofenadine, gelatin, glycerol and sorbitol are present in the following ratios with respect to water:
- the ratio of gelatin is from about 0.3 - 0.4:1 by weight,
- the ratio of glycerol is from about 0.7 - 1.3:1 by weight,
- the ratio of sorbitol is from about 0.4 - 1.4:1 by weight.

According to a preferred embodiment of the invention, when the at least one pharmaceutically active ingredient is a combination of magnesium and aluminum hydroxides, with respect to water:
- the ratio of gelatin is from about 0.2 - 0.35:1 by weight,
- the ratio of glycerol is from about 1.2 - 1.5:1 by weight,
- the ratio of sorbitol is from about 0.2:1 - 0.4:1 by weight.

According to a preferred embodiment of the invention, when the at least one pharmaceutically active ingredient is a combination of primula and thyme plant extracts, with respect to water:
- the ratio of gelatin is from about 0.3 - 0.7:1 by weight,
- the ratio of glycerol is from about 1.3 - 2.9:1 by weight,
- the ratio of sorbitol is from about 0.3 - 0.6:1 by weight.

According to a preferred embodiment of the invention, when at least one pharmaceutically active ingredient is Acetaminophen, or pharmaceutically acceptable salts thereof, the chewable oral gum pharmaceutical formulation comprises:
- the ratio of gelatin to water is from about 0.1:1 to about 0.4:1 by weight,
- the ratio of glycerol to water is from about 1.2:1 to about 1.6:1 by weight,
- the ratio of sorbitol to water is from about 0.15:1 to about 0.3:1 by weight.

### Gelatin:

One advantage of the chewable oral gum pharmaceutical formulation of the present invention is that the gelling agent is gelatin, which dissolves at about 30-35°C. Higher temperatures, necessary for some other gelling agents including pectin and pectin derivatives, may cause degradation of heat sensitive pharmaceutical ingredients.

Any suitable type of gelatin may be present in the formulation according to the invention. For example, the gelatin may be animal-derived gelatin, chemically-modified gelatin, physically-modified gelatin, and combinations thereof. Animal-derived gelatin may be derived from any suitable source such as, for example, pigskin or bovine bone or bovine hide or fish skin. Alternatively, the gelatin may be hydrolyzed gelatin. Hydrolyzed gelatin is also commonly known as hydrolyzed collagen, collagen hydrolysate, and collagen peptide.

A "Bloom value" is often used to characterize the gel strength of a gelatin. According to one embodiment of the invention, the gelatin has a Bloom value of from 150 to 280, for example, 160, or 180 or 200, or 220 or 250, preferably 120-200.

Examples of suitable gelatins are those sold under the trade names Rousselot 160 LB8 (of Bovine origin) Rousselot 180 PS (pig skin origin), and gelatin 75 (also pig origin), available from Rousselot (Netherlands).

According to a preferred embodiment, the gelatin used is Gelatin 160 LB8, Gelatin 75 (pork), or a mixture thereof.

According to one embodiment of the invention, the amount of gelatin in the formulation may be about 4-12% w/w.

According to a preferred embodiment of the invention, when the at least one pharmaceutically active ingredient is a combination of primula and thyme plant extracts, the amount of gelatin in the formulation may be about 8-14%, preferably 10-12% w/w.

According to a preferred embodiment of the invention, when the at least one pharmaceutically active ingredient is a combination of magnesium and aluminum hydroxides, the amount of gelatin in the formulation may be about 6-14% w/w, preferably, 8-12% w/w.

According to a preferred embodiment of the invention, when the at least one pharmaceutically active ingredient is fexofenadine, or pharmaceutically acceptable salts thereof, the amount of gelatin in the formulation may be about 6-12% w/w, preferably 8-10% w/w.

### Glycerol:

The oral gum formulation according to the invention contains glycerol, also commonly called glycerin. Glycerol is used as a humectant and sweetener.

Glycerol is usually added to the formulation of the invention as the pure liquid form, which is readily available commercially.

According to a preferred embodiment of the invention, when the at least one pharmaceutically active ingredient is fexofenadine, or pharmaceutically acceptable salts thereof, the ratio of glycerol is from about 0.7-1.3:1 by weight with respect to water.

According to a preferred embodiment of the invention, when the at least one pharmaceutically active ingredient is a combination of magnesium and aluminum hydroxides, the ratio of glycerol is from about 1.2-1.5:1 by weight with respect to water.

According to a preferred embodiment of the invention, when the at least one pharmaceutically active ingredient is a combination of primula and thyme plant extracts, the ratio of glycerol is from about 1.3-2.9:1 by weight with respect to water.

According to one embodiment of the invention, the amount of glycerol in the formulation may be about 16-60% w/w.

According to a preferred embodiment of the invention, when the at least one pharmaceutically active ingredient is a combination of primula and thyme plant extracts, the amount of glycerol in the formulation may be about 35-60%, preferably 40-55% w/w.

According to a preferred embodiment of the invention, when the at least one pharmaceutically active ingredient is a combination of magnesium and aluminum hydroxides, the amount of glycerol in the formulation may be about 30-40% w/w, preferably, 32-38% w/w.

According to a preferred embodiment of the invention, when the at least one pharmaceutically active ingredient is fexofenadine, or pharmaceutically acceptable salts thereof, the amount of glycerol in the formulation may be about 16-40% w/w, preferably 18-35% w/w.

### Sorbitol:

Sorbitol, a polyol sugar, also known as E420, is present in the chewable oral gum pharmaceutical formulation, according to the invention. The presence of sorbitol avoids the use of sucrose in the formulations, sucrose being caloric and cariogenic. One particular advantage of the formulation according to the invention is that said formulation is sugar free.

Sorbitol may be added to said formulation in a dry or liquid (as a solution) form. For example, it may be added as a 70% v/v aqueous solution, which is commercially available. In that case, the equivalent amount of dry sorbitol is used to calculate the amount of sorbitol solution to add to the formulation, the amount of water in the 70% sorbitol solution being taken into account in the calculation of the amount of total water in the formulation.

Generally, sorbitol is present in the formulation of the present invention in a ratio of 0.15 - 1.45:1 by weight, with respect to water.

According to one embodiment of the invention, when fexofenadine, or a pharmaceutically acceptable salt thereof, is the active ingredient, sorbitol is present in the formulation in a ratio of 0.40 - 1.40:1 by weight, with respect to water.

According to one embodiment of the invention, when the active ingredient is a combination of magnesium and aluminum hydroxides, the ratio of sorbitol is from about 0.2:1 - 0.4:1 by weight, with respect to water.

According to one embodiment of the invention when the active ingredient is a combination of primula and thyme plant extracts, sorbitol is present in the formulation of the present invention in a ratio of 0.3 - 0.6:1 by weight, with respect to water.

According to one embodiment of the invention, the amount of sorbitol in the formulation may be about 4% -30% w/w.

According to a preferred embodiment of the invention, when the at least one pharmaceutically active ingredient is a combination of primula and thyme plant extracts, the amount of sorbitol in the formulation may be about 8-15%, preferably 10-12% w/w.

According to a preferred embodiment of the invention, when the at least one pharmaceutically active ingredient is a combination of magnesium and aluminum hydroxides, the amount of sorbitol in the formulation may be about 4.5-10 % w/w, preferably, 5-6 % w/w.

According to a preferred embodiment of the invention, when the at least one pharmaceutically active ingredient is fexofenadine, or pharmaceutically acceptable salts thereof, the amount of sorbitol in the formulation may be about 8-34 % w/w, preferably 12-32 % w/w.

### Water / water activity:

Preferably, water introduced into the formulation according to the invention is purified water according to the U.S. and E.U. pharmacopeias. Water is used to hydrate gelatin, and/or dissolve excipients and active pharmaceutical ingredients.

Generally, the formulation according to the invention has a water activity of less than 0.75.

The « water activity » represents the water availability (i.e., free water) available to microorganisms within a sample for growth. The water activity may be measured by methods known in the art. For example, as suitable equipment for measuring water activity, one may cite Hygrolab C1 (used with for example, a HygroClip 2 probe), available from Rotronic (France).

Generally, the water activity is less than 0.75, preferably, less than 0.70 and more preferably less than 0.65. This low value for the water activity is particularly advantageous because it renders the addition of preservatives, such as parabens to the formulation, unnecessary.

### Thickening agents:

According to an embodiment of the invention, thickening agents such as Xanthan gum, gellan gum (including high acyl and low acyl grades and combinations thereof), tragacanth gum and arabic gum may be included in limited amounts in the formulations, individually or as a mixture. This may be the case when the active ingredient is one that has an unacceptable taste, and requires the presence of further agents in addition to traditional taste masking agent. Similarly, additional gelling agents such as carrageenans, alginates, carboxymethylcellulose and starch derivatives may be present in limited amounts insofar as they may have a melting temperature of less than 60°C. By "limited amounts" with respect to thickening agents and additional gelling agents, it is meant less than 2% w/w. Preferred amounts are 0.2% - 1.5% w/w, more preferably, about 0.2% - 0.50% w/w, and most preferably about 0.24% - 0.27% w/w.

According to an embodiment of the invention, when the active pharmaceutical ingredient is Acetaminophen, or pharmaceutically acceptable salts thereof, the chewable OG formulation may contain xanthan gum in an amount of about 0.2% - 1.1% w/w, preferably about 0.2% - 1.03% w/w, and most preferably about 0.24% - 0.27% w/w.

### Surfactant:

According to certain embodiments of the invention, the formulation may comprise at least one surfactant, in a limited amount. For example, in the case of liquid active ingredients, for example liquid plant extracts, the combination of primula and thyme plant extracts, or vitamins, the presence of surfactant is advantageous.

When present, the surfactant may be a non-ionic, cationic, amphoteric or anionic surfactant. Preferably, a non-ionic surfactant is used. Advantageously, the surfactant is chosen among the group consisting of: ethylene propylene, polyoxyethylene (20), oxide copolymers, such as poloxamers those sold under the trade names Synperonics®, Pluronics®, and Kollipho®, polysorbates, such as those sold under the trade name TWEEN (supplied by Croda Americas, Inc.). Preferably, the surfactant is sorbitan monooleate (or polysorbate 80), such as sold under the trade name TWEEN 80® (supplied by Croda Americas, Inc. USA).

When the active pharmaceutical ingredients are a combination of thyme extract and primula extract, the chewable oral gum pharmaceutical formulation may contain, for example, TWEEN 80, in an amount of from about 0.1% - 0.5% w/w, preferably from about 0.1% - 0.4% w/w and more preferably from about 0.1% - 0.3% w/w.

According to an embodiment of the invention, when the active pharmaceutical ingredient is Acetaminophen, or pharmaceutically acceptable salts thereof, the chewable oral gum pharmaceutical formulation may contain a surfactant, such as TWEEN 80® in an amount of from about 0.1% w/w to about 0.4% w/w, preferably from about 0.1% w/w to about 0.3% w/w and more preferably from about 0.1% w/w to about 0.2% w/w.

Without being bound by theory, it is believed that the presence of the surfactant further aids (as well as the composition of the oral gum itself) in demolding the gelled formulation.

Plasticizers such as polyethylene glycol, for example 400 (PEG 400), and polyethylene glycol 4000 (PEG 4000) may also be present in the formulation in limited amounts (2% to 8%).

According to one embodiment of the invention, when the active pharmaceutical ingredient is fexofenadine, in the form of fexofenadine HCI, the chewable oral gum pharmaceutical formulation may contain an additional agent such as a PEG, preferably PEG 400 to increase the bioavailability of the Fexofenadine HCI. PEG may be present in an amount of from about 0% - 6% w/w.

In the case of fexofenadine, other agents known to increase the bioavailability of the active substance may be included in the formulation. For example, ionic surfactants, such as sodium cholate or sodium taurocholate are known to increase the intestinal permeability of fexofenadine HCI. P-glycoprotein inhibitors are also known to increase the bioavailability of fexofenadine HCI (see US 6451815) and may be added in limited amounts. P-glycoprotein inhibitors include, for example, water soluble vitamin E, polyethylene glycol, poloxamers including Pluronic F-68, Polyethylene oxide, polyoxyethylene castor oil derivatives including Cremophor EL and Cremophor RH 40, Chrysin, (+)-Taxifolin, Naringenin, Diosmin, Quercetin, and the like. Suitable amounts of these P-glycoprotein inhibitors that may be added to the formulation are detailed in US 6451815 (see col. 7, l. 61 to col. 8, 1.24).

Silica may also be added in limited amounts to the chewable OG formulation. The inventors have found that the presence of silica in the formulation may further aid in the demolding of gelled formulation. According to one embodiment of the invention, when the active pharmaceutical ingredient is fexofenadine, or pharmaceutically acceptable salts thereof, the chewable oral gum pharmaceutical formulation may contain silica in an amount of from about 4% -10 % w/w, preferably from about 6 % - 8% w/w. Suitable forms of silica are supplied, for example, under the name of "Syloid" by Grace (Maryland, USA).

### Sugar substitutes, flavoring agents, coloring agents:

Additionally, the chewable oral gum pharmaceutical formulation according to the invention may comprise at least one another ingredient including for example, additional sugar substitutes, flavoring agents, and/or coloring agents. The latter agents may be added in amounts known to the skilled person, for example, less than 0.4% w/w.

By additional sugar substitute, it is meant a food additive that replicates the taste of sugar. Preferably, said sugar substitutes are artificial sweeteners such as sucralose, isomalt, isomalt 990, isomalt 721, saccharine and/or potassium acesulfame. The additional sugar substitutes may be added in amounts known to the skilled person, for example less than 2% w/w, preferably, less than 0.4% w/w.

The flavoring agents are preferably selected in the group consisting of lime, lemon, strawberry, apple, menthol, honey, ginger, pineapple and tropical.

By coloring agents, it is meant dyes, lakes and/or opacifying agents. Examples of such coloring agents are red iron oxide, yellow iron oxide, TiO₂, carmine E120, FD&C blue no. 1 Aluminium Lake, beta carotene E160a etc.

### pH adjusting agent:

To facilitate gelling of the formulation, and to achieve a desirable pH for the final chewable oral gum pharmaceutical formulation, the pH of the formulation may be adjusted using suitable pH adjusting agents. The final formulation usually has a pH of about 3.5 to 8. As non-limiting examples of acid pH adjusting agents, one may cite citric acid, tartaric acid, phosphoric acid and lactic acid, sodium hydroxide, sodium dihydrogen phosphate, monosodium dihydrogen orthophosphate and monosodium dihydrogen monophosphate.

Preferably, if the active ingredient of said formulation is fexofenadine, the pH is adjusted with sodium hydroxide, sodium dihydrogen phosphate, monosodium dihydrogen orthophosphate and monosodium dihydrogen monophosphate.

According to one embodiment of the invention, the pH of the formulation is adjusted to a value between 6.5 and 7.5, for example, in the case when fexofenadine is the active ingredient.

### Administration of the oral gum formulation:

It may be noted that once gelled, the formulation according to the invention is in the form of a chewable solid. The solid unit may be in any shape suitable for administration to the patient. For example, for administration to children, the chewable oral gum pharmaceutical formulation may be in a shape of an animal or character or object. For people having few or no teeth, said chewable formulation may also be sucked.

This is of particular interest for a pharmaceutical formulation destined to be administered to children. Additionally, it is advantageous that the pharmaceutical formulation, especially for administration to children, has a pleasant taste. Therefore,
- the taste of the formulation may be modified by the addition of suitable sugar substitute(s) and flavoring agent(s), and
- coloring agent(s) may render the chewable formulation more attractive.

These features contribute positively to patient compliance.

The chewable oral gum pharmaceutical formulation of the invention, under solid form, is generally dosed into individual units. Preferably, each unit comprises a dose of drug substance suitable for an adult, or suitable for a child.

### Process:

The present invention further relates to a process for the preparation of the above-described chewable oral gum pharmaceutical formulation. The inventors have developed a continuous pharmaceutical manufacturing process that meets pharmaceutical regulatory and quality guideline requirements. The blister pack thermoforming, formulation, preparation, heating and pouring, blister sealing, blister cooling and, optionally, cutting are preferably carried out in the one continuous production line.

Figure 1A shows a schematic representation of one embodiment of the process for OG production, showing in particular the continuous production line.

By "continuous" production line is meant a production line, in which all of the steps to produce the packaged (and thus closed and protected from contamination) final product from the initial product preparation step to the final blister sealing step, or optionally, the final blister package cutting step, are carried out in the same line. Thus, generally, the heated formulation is poured into newly thermoformed blisters, which are cooled and sealed. According to a preferable embodiment of the invention, the filled blisters are sealed first and then cooled. Alternatively, the filled blisters are sealed and cooled at the same time. According to an embodiment of the invention, the filled blisters are cooled and then sealed.

Optionally, in the same production line, the blister package is then cut into units of a suitable number, for example into units of 6, or 12, or 24 for packaging in a secondary package.

The formulation pouring step is controlled by measuring the amount of formulation dispensed in to the blister, preferably using a laser, thereby ensuring correct formulation dosing. Because the process is continuous, risk of contamination of the oral gums and of the machinery that may be introduced in the (manual) transfer of the formulation from one functional unit to another is minimized.

Thus, the production line is preferably modular assembly of functional units or stations that successively carries out the steps of the continuous manufacturing process. Generally, the production line comprises at least one blister thermoforming unit, a formulation or preparation unit, a pouring or filling unit, a sealing unit wherein a film is continuously placed to cover the filled blisters, a sealing unit, a cooling unit and optionally a cutting unit.

The continuous production line has been especially conceived to produce the chewable oral gum pharmaceutical formulation described previously.

The process to prepare the above described chewable oral gum pharmaceutical formulation comprises at least the following steps:
(i) adding the glycerol,
(ii) adding the sorbitol,
(iii) adding the water,
(iv) adding the gelatin,
(v) heating the mixture from about 45°C to 75°C,
(vi) adding the at least one pharmaceutically active ingredient,
(vii) optionally, adding the sugar substitute(s) and/or the coloring agent(s) and/or the flavoring agent(s),
(viii) removing air bubbles from the formulation,
(ix) pouring the formulation into (optionally newly) thermoformed blisters to a predefined target liquid level in the blister cavity,
(x) sealing the blister pack containing the formulation with a film/sheet/cover,
(xi) cooling the filled blister pack,
(xii) optionally, cutting the blister pack into units of suitable size for packaging.

According to a preferred embodiment of the invention, the pouring step (ix) is carried out using an automated filler unit connected to a laser operated measuring unit. The laser measures the level of the poured formulations in the blister. The amount of liquid poured into a given blister is adjusted as a function of the level of the liquid formulation measured in the preceding blister.

### Steps (i) to (viii):

Generally, steps (i) to (vi) may be performed in any order, depending on the active ingredients to be included in the formulation. Where appropriate, the process steps (i) to (vi) may be performed as a series of sub-steps with partial addition of reagents at different times in the process.

Steps (i) - (viii) are generally carried out with mixing, using suitable mixing speeds known to the skilled person. The choice of mixing speeds varies with tank volume, mixture viscosity and ingredient presentation and other factors known to the skilled person. Generally, a solid ingredient may be pulverant or granular, and a pulverant ingredient may require a higher mixing speed than one that is granular. Similarly, a liquid ingredient may be fluid or viscous; a viscous ingredient may require slower mixing speeds compared to a more fluid one, etc.

Grinding may also be needed when adding certain ingredients. For example, grinding is usually carried out when fexofenadine and red iron oxide are added in the preparation of the fexofenadine OG formulation. The skilled person knows when and how grinding should be used.

Heating step (v) may be applied to any of the steps (i) to (ix) depending on the physicochemical characteristics of the active ingredients to be used.

For example, for magnesium hydroxide and aluminum hydroxide, the heating temperature ranges from about 65°C to about 75°C.

For example, for fexofenadine, the heating temperature ranges from about 60°C to about 65°C.

For example, for Acetaminophen, the heating temperature ranges from about 55°C to about 65°C, preferably 60°C.

However, for heat sensitive active ingredients, for example plant extracts such as thyme extract and primula extract, the mixture containing these ingredients is generally kept at a temperature below 55°C, preferably between 45 °C and 55°C.

According to an embodiment of the invention, one or more pH adjustment steps may be carried out at any appropriate point in the process.

According to some embodiments of the invention, one or more de-caking steps may be added to remove any clumps formed by insoluble ingredients and/or excipients from the formulation mixture. This may be the case, for example, for the preparation of formulations containing magnesium hydroxide and aluminium hydroxide as the active ingredient.

According to an embodiment of the invention, the preparation of the pharmaceutical formulation mixture (steps (i) to (viii)) may be carried out in one container or tank. Suitable tanks for the preparation of pharmaceutical formulations, are known to the skilled person.

According to an embodiment of the invention, the preparation of the pharmaceutical formulation mixture (steps (i) to (viii)) may be carried out in two or more containers or tanks.

According to an embodiment of the invention, in which the active pharmaceutical ingredient is fexofenadine, one tank or two tanks may be used. A description of steps (i) to (viii) of a typical "one tank process" (in which steps (i) to (viii) are carried out in one tank), using fexofenadine as an example, is given below (see also Example 1).

Fexofenadine exists as a zwitterion in aqueous media at physiological pH. The drug has two ionization groups corresponding to free carboxylic group on the side chain and the substituted ring nitrogen, contributing to a pKa value of 4.25 and 9.53, respectively. An aqueous solution of Fexofenadine HCI is acidic and the pH of a 0.1% (w/v) aqueous solution falls in the range of 3.2-3.3. Therefore, to dissolve Fexofenadine HCI, preferably, the pH of the water should be adjusted. This pH adjustment step facilitates the dissolution of the zwitterion. The pH adjustment may be carried out before or at the same time as the addition of the fexofenadine HCI. According to one embodiment of the invention, the pH of the water in which Fexofenadine HCI is to be dissolved is adjusted before addition of fexofenadine. Thus, according to one embodiment of the invention when fexofenadine is the active ingredient, the pH is adjusted to about 6.5 - 7.5 using a suitable buffer, for example sodium phosphate. Then, the pH may be raised to about 12-14, preferably 13, using, for example NaOH. Thus, following the addition of suitable buffers (e.g. phosphate and NaOH), the fexofenadine is added. The pH of the mixture is generally maintained at 6.5-7.5. Optionally, coloring agents may be added to the solution.

Typically, no specific pH adjustment is necessary for the combination of aluminum hydroxide and magnesium hydroxide.

Typically, no specific pH adjustment is necessary for the thyme and primula plant extract.

Generally, the degassing step (viii) in which any air bubbles are removed may be carried out concomitantly with any of steps (i) - (vii).

According to an embodiment of the invention, steps (vii) and (viii) may be carried out concomitantly. According to an embodiment of the invention, after partial degassing to remove air bubbles, sorbitol, optionally, sweeteners, and glycerol may be added with mixing and heating.

Mixing may be generally carried out at 20-90 rpm. The skilled person understands that the mixing speed may vary with the equipment being used, for example, whether using an internal and external paddle or internal paddle alone. The mixing speed may vary with the physical state of the mixture, with higher mixing speeds being permitted when the mixture becomes more homogeneous and less viscous; the viscosity depends on the temperature of the mixture as well as the nature of the ingredients added. The mixing speed may thus vary according to the physical nature of the ingredients used in the process. The examples in the Examples section below illustrate a number of different embodiments of the invention according to the different active ingredients used.

Preferably, the mixing is carried out under vacuum. Any suitable mixing equipment known to the skilled person may be used.

Preferably, the gelatin is added slowly, with mixing, preferably during a period of minimum two hours. Further degassing may be carried out. This pharmaceutical formulation may be used to form the oral gums in step (ix).

According to an embodiment of the invention, for example, when the active ingredient is a combination of aluminium hydroxide and magnesium hydroxide, the water, glycerol and sorbitol may be added and mixed with heating, at 55-75°C, preferably, 70°C. The active ingredient(s) may then be added, and optionally, other excipients, sweeteners, coloring agents etc.

According to an embodiment of the invention, for example, when the active ingredient is a mixture of aluminium hydroxide and magnesium hydroxide, a de-caking, or milling step is advantageous to eliminate or prevent lumps of active ingredients in the pharmaceutical formulation. A clod breaker is carried out by any suitable means, including activating a clod breaker near the bottom of the tank.

Typically, gelatin may then be added and let hydrate (for example, for a period of at least two hours). Other components, for example flavoring and/or coloring agents, may then be added. This pharmaceutical formulation may be used to form the oral gums in step (ix).

Other examples of active ingredients that may be used to prepare gums in a similar way to that described for the mixture of aluminum hydroxide and magnesium hydroxide are calcium carbonate and magnesium citrate.

According to an embodiment of the invention, the preparation of the pharmaceutical formulation mixture (steps (i) to (viii)) may be carried out in more than one tanks, the principal tank, in which the primary blend of the formulation is prepared, being connected to one or more secondary tanks that feed into the principal tank.

According to one embodiment of the invention, water and/or other components may be added to this/these secondary tank(s) to pre-dissolve certain water soluble components, for example, gelatin, and/or to form homogenous mixtures, and/or to mix heat sensitive ingredients, before they are added to the principal tank. For example, flavoring and/or coloring agents, may thus be added from the secondary tank.

According to one embodiment of the invention, in which the active ingredient is heat-sensitive, or requires different solubilizing or dispersion conditions to some or all of the other ingredients in the formulation, the active ingredient may be dissolved or dispersed in a secondary tank, that feeds into the principal tank.

As a heat-sensitive ingredient, we may cite as an example, thyme extract and/or primula extract. Generally, this ingredient should not be heated above 55°C.

According to one embodiment of the invention, in which the active ingredients are heat-sensitive, for example, when the active ingredients are thyme extract and/or primula extract, a primary blend of components may be prepared by mixing glycerol, sorbitol and gelatin at about 55-75°C, preferably about 60°C to 70°C, until the mixture is homogeneous. In a second tank, a secondary blend containing water and the heat sensitive ingredients may be prepared at room temperature (19°C - 25°C). Optionally, sweetener(s) such as sucralose, aroma and/or flavoring may be added to the secondary blend or to the primary blend. The secondary blend may be mixed, advantageously until homogeneous. Mixing may be carried out at a suitable speed to ensure that the mixture becomes homogenous, however, without damaging any active ingredients that may be sensitive to physical stress. Mixing may be carried out at, for example, 300-350 rpm. The temperature of the primary blend is generally allowed to decrease to a suitable temperature, preferably about 45 to 55°C, more preferably about 50°C. Then, the secondary blend may be added to the primary blend (or vice versa), and mixed. The resulting pharmaceutical formulation may be used to form the oral gums in step (ix).

According to one embodiment of the invention, the heating temperature of formulation at heating step (v) and/or pouring step (ix) is about 45°C to about 55°C.

For example, in embodiments in which the active pharmaceutical ingredient of the formulation according to the invention is Acetaminophen, or pharmaceutically acceptable salts thereof, the formulation may be typically prepared in two tanks. A primary blend may be prepared in the principal tank, by combining Acetaminophen, water, glycerol and sorbitol and optionally other ingredients such as thickeners, etc. A secondary blend comprising water and gelatin may be mixed, and then combined with the primary blend, to form the pharmaceutical formulation to be used to form the oral gums in step (ix). Preferably, micronized Acetaminophen is used.

Further examples of active ingredients that require separate treatment in a separate (for example, second or third etc.) tank and the OG formulation of which may be prepared in a similar way to that described for thyme extract and primula extract are other active substances, including plant extracts, requiring conditions to be dissolved, or suspended or emulsified that are incompatible with the conditions in the main tank.

According to one embodiment of the invention, the active ingredient(s) may be added to the formulation mixture, as late in the process as is possible while ensuring the ingredients homogeneous dispersion or solution. We may cite for example, active ingredients that are unstable to heat or oxygen, such as vitamins, for example, Vitamin C.

### Viscosity:

Advantageously, when the formulation is heated for pouring (step ix), it has a viscosity that allows it to be poured without sticking in the nozzles of the filling units.

The measurement of viscosity is well known to those skilled in the art. In the present case, it may be carried out using a Brookfield Rheometer DV3Textra using a small sample adapter to be able to keep the temperature of the mass. The following parameters (parameter set A) for the latter apparatus, for example, may be used for the measurement: spindle: 29, torque: 50 rpm, torsion couple 40.3%, temperature 50°C, end time: 2min 30sec.

According to one embodiment of the invention, when the active substance is a combination of primula and thyme plant extracts, the viscosity of the mixture at pouring, as measured according to the above parameter set A, is about 750-760 mPAs, preferably, 756 mPas.

The following parameters (parameter set B) for example, may be used in measuring viscosity: mobile No.6, temperature at about 55.5°C, torque at about 5 rpm and torsion couple at about 16.3%.

According to one embodiment of the invention, when the active substance is fexofenadine, the viscosity of the mixture at pouring, as measured according to the parameter set B, may be from about 32 600 cP to 34 200 cP.

The viscosity measurement may be carried out using a Brookfield Rheometer DV2T with following parameters (parameter set C): Mobile 6, RV06, torque at about 50 rpm, torsion couple 13.7%, temperature 60°C.

According to one embodiment of the invention, when the active substance is the combination of magnesium hydroxide and aluminum hydroxide, the viscosity of the mixture at pouring mixture, may be from about 2700 to 2750, preferably 2740 cP, as measured according to parameter set C.

The viscosity measurement may be carried out using a Brookfield Rheometer DV2T with following parameters (parameter set D): Mobile 5, torque at about 50 rpm, RV05, torsion couple 19%, temperature 60°C.

According to one embodiment of the invention, when the active substance is the combination of magnesium hydroxide and aluminum hydroxide, the viscosity of the mixture at pouring mixture, may be from about 1500 to 1600, preferably 1520 cP, as measured according to parameter set D.

The presence of surfactant in the formulation, such as, for example, TWEEN 80®, may help reduce the risk of the formulation sticking in the filler unit nozzles at the pouring step.

Before proceeding to step (ix), preferably, air bubbles are preferably eliminated from the mixture. Preferably, air bubbles are eliminated under vacuum or under stop stirring. Elimination of air bubbles may be carried out more than once during the process.

### Step (ix):

Generally, the pharmaceutical formulation is poured directly into blister pack cavities. Blister pack cavities serve as a mold for the formulation.

According to one embodiment of the invention, the plastic film used to form the blister pack cavities is a deformable film available in the pharmaceutical packaging field which is adapted to form blisters. According to one embodiment of the invention, the blister of the individually dosed administration forms is selected from PVC (polyvinyl chloride), PVDC (polyvinylidene chloride), PP (polypropylene), or laminates such as PVC-PVDC-PVC. For example, a PVDC layer can have a density of 120g/m² and PVC layer can have a thickness of 127 micrometers. Examples of commercially available materials, suitable for thermoforming blister packs are Pentapharm® Alfoil® range by Klöckner Pentaplast (Germany), for example Pentapharm® Alfoil® E S03, Pentapharm® Alfoil® XC SG and also the Pentapharm® ACLAR® range, for example, Aclar 300 S03, also available from Klöckner Pentaplast (Germany).

Generally, the blister pack cavities are made using a thermoforming process. The thermoformed cavities are moved directly to the pouring unit in a continual process.

According to one embodiment of the invention, a guide rail allows movement of the thermoformed blisters under the filling unit. Generally, the pharmaceutical formulation is poured when the thermoformed blister has stopped moving.

Generally, the formulation obtained in step (viii) is poured into thermoformed blisters using an automated filler unit, which is connected to a measuring unit that measures the level of the formulation poured, with respect to the top of the blister opening where the covering film is placed. The amount of liquid poured into a given blister is adjusted as a function of the level of the liquid formulation measured in the preceding blister.

The pouring step is generally carried out at a temperature that is high enough that the formulation is in a liquid state, preferably at a viscosity wherein the formulation does not stick to the filling unit nozzles. Herein, the "pouring temperature" is defined as the temperature of liquid formulation when it is poured at step (ix).

The correct pouring temperature ensures the homogeneous distribution of formulation mixture which should be sufficiently fluid in the blister to obtain a flat surface. If pouring temperature is too high (for example, greater than 75°C), a plastic blister can melt and heat sensitive ingredients in the formulation lose biological activity. A lower pouring temperature (for example, 45°C to 65°C) is advantageous as it saves heating time and energy in the manufacturing process.

According to an embodiment of the invention, if active ingredients are plant extracts and/or vitamins, pouring temperature may be in the range of 45 - 55°C.

If on the other hand, the pouring temperature is too low (for example, below 40°C), the surface of the gelled formulation may be not smooth, or said formulation can become too hard and become stuck in the nozzle of the dosing unit.

The inventors have found that, generally, for active ingredients that are not heat sensitive, a pouring temperature 40- 70°C may be used.

According to an embodiment of the invention, in which the active ingredients are a combination of magnesium hydroxide and aluminum hydroxide, the pouring temperature may be in the range of 55°- 70°C, preferably 60°C.

According to one embodiment of the invention, in which active ingredient is Acetaminophen, the pouring temperature may be in the range from about 40°C to about 50°C, preferably, 42-45°C.

### Steps (ix), (x) and (xi):

Figure 1B shows a representation of the filling unit (5) (also known as dosing unit) and the laser measuring unit.

In pouring step (ix), the filling unit (5) is controlled by a measurement unit (6), preferably a measuring unit (6) operating a laser (6a). The measuring unit (6) is typically located adjacent to the filling unit (5). The filling unit and measuring unit may be preferably located in, or, adjacent to a cooling unit (7).

The cooling serves to at least partially harden the formulation before the sealing step (x) so that irregular forms, for example, "waves" due to the movement of the blisters, do not form on the formulation surface, which should be flat and even when the formulation hardens.

Thus, generally, the cooling step (xi) of the process may be carried out before blister sealing, or, after blister sealing, or both before and after sealing the blister packs.

Typically, the cooling may occur for a period of about 15 seconds to 2 minutes, with temperatures at between about 0°C and 10°C. The cooling unit (7) may be a laminar air flow chamber. Typically, laminar air flow over the blisters serves not only to cool the formulation, but also to avoid external contamination of the formulation by dust or other contaminants, as it is being poured into the blisters (3), or, after it is poured into the blisters but before the blister is sealed.

According to one embodiment of the invention, the laminar flow unit may be integrated in the process line. According to one embodiment of the invention, the laminar flow unit may be a separate mobile unit that may be displaced at various positions along the process line.

According to one embodiment of the invention, the filled blisters are cooled for a period of about 30 seconds to 1 minute, preferably for 40 to 45 seconds. Generally, the cooling temperature is between about 0°C and 5°C, preferably 3-5 °C. Thus, if a laminar flow unit is used for cooling, the cooling temperature refers to the temperature of the air in the air flow.

According to one embodiment of the invention, the filled blisters are cooled for a period of about 15 seconds to 90 seconds, preferably, for 35 to 45 seconds, with the air temperature at about 8°C.

The level of the formulation poured into the blister cavity may be measured using a laser detection cell (6). The laser beam (6a) is generally aimed at the center of the blister cavity (or alveolus) (3). The laser measures the depth of the empty blister cavity, and the height of the level of the formulation deposited in the blister after pouring. Based on the signal received from the measuring unit, the distance between the level of the poured formulation and the top of the blister pack (where the sealing film will be placed) is calculated. Preferably, this distance should be about 1.0 to 2.5 mm, more preferably 1.8 to 2.2 mm, most preferably, 2.0 mm. This distance is the "predefined target distance".

By filling the blister with the formulation to this predefined target distance, the inventors have found that the formulation does not splash outside the cavity, before sealing step (x), and does not splash onto the sealing film during, or after sealing step (x). Therefore, the correct amount of formulation is poured into the blisters, ensuring correct dosage amounts in each blister.

Thus, the measuring unit (6) transmits the measured distance in the blister to the pouring unit. The pouring unit (5) may then operate a compensation or regulation of the filling level, preferably by faster opening or closing of the filling injectors or filling nozzles (4). The accurate dosage of the formulation generally depends on the opening time of the valve and the diameter of the injector (4) of the pouring unit (5). Thus, the speed of the filling injector opening and closing is regulated, preferably automatically, as a function of the distance measured by the laser.

According to one embodiment of the invention, after three consecutive pouring adjustments failing to result in the formulation being poured to the predefined target distance, the blisters are considered to be outside predefined tolerance limits, and are evacuated from the production line into a receiving tank.

According to an embodiment of the invention, the measuring unit may contain one or more laser beams. According to an embodiment of the invention, the measuring unit may contain up to the same number of laser beams as blister cavities to be filled. For example, twelve lasers beam may be aimed at the center of twelve blister cavities, that are filled with twelve injectors. For example, six lasers beam may be aimed at the center of six blister cavities, that are filled with six injectors.

The filled blister cavities are sealed with a suitable film. According to one embodiment of the invention, the cavities are sealed with any suitable film, for example, an aluminum film, or an aluminum with a paper layer film, or an aluminum and paper and a polyethylene terephthalate (PET) film, or an aluminium and HSL (Heat Seal Lacquer) film, or a PET and aluminium and HSL film or an aluminium and HSL film. Examples of materials that may be used for sealing the blisters are available from Amcor (Switzerland) under the following trade names "ACE-100-700" (Amcor standard lidding foil: Alu 20µ and HSL), "DB034 TDSP0057-E" (Amcor peelable lidding foil: PET23µ/Alu20µ and HSL), "DB112 TDSP0313-E" (Amcor peelable lidding foil: Alu 38µ soft and HSL) and "DB089 TDSP0257-E" (Amcor peelable lidding foil: Paper50g/PET12µ/Alu20µ and HSL).

Typically, the sealing film is fed from a sealing film coil (8). The sealing step (x) is carried out using any suitable equipment, known to the skilled person, for example a sealing plate (9).

According to an embodiment of the invention, after sealing step (x), the blisters are further cooled so that the formulation hardens to form a gum. According to one embodiment of the invention, the blisters circulate continuously into a cooling station. The cooling temperature may be generally within the range of 8°-14°C. Preferably, the cooling is carried out with a set of cooling air jets incorporated into plates cooled by a water solution circuit.

Cooling may also be operated at ambient temperature, for longer periods of time. The skilled person knows how to adjust the cooling temperature and time to achieve a hard oral gum that has an even surface.

After cooling, according to one embodiment of the invention, one may optionally cut the blister packs into units of suitable size for packaging using suitable cutting means (10), known to the skilled person.

The invention also relates to a chewable oral gum pharmaceutical formulation described above, wherein it is obtainable using the described process.

### EXAMPLES

The examples are not limiting and merely illustrate the present invention.

### Example 1: Oral gum comprising fexofenadine (unit weight of 180 mg)

### Formulation PCB3860:

| **Ingredients** | **% wt per unit** |
|---|---|
| Purified water | 26.42 |
| NaOH | 0.78 |
| NaH₂PO₄ H₂O | 0.29 |
| Na2_{H}PO₄ 2H₂O | 0.08 |
| Red iron oxide | 0.01 |
| Fexofenadine HCl | 8.51 |
| Sorbitol 70% | 23.99 |
| Sucralose | 0.24 |
| Glycerin 95% | 18.94 |
| Syloid (Silica) | 6.98 |
| Gelatin 160 LB8 | 9.00 |
| PEG 4000 | 5.00 |
| Total | 100.00 |

### Formulation PCB4375:

| **Ingredients** | **% wt** |
|---|---|
| Purified water | 26.42 |
| NaOH | 0.78 |
| NaH₂PO₄ H₂O | 0.29 |
| Na₂HPO₄ 2H₂O | 0.08 |
| Red iron oxide | 0.01 |
| Fexofenadine HCl | 8.51 |
| Sorbitol | 23.99 |
| Sucralose | 0.24 |
| Glycerin 95% | 18.94 |
| Syloid | 6.98 |
| Gelatin 160 LB8 | 5.00 |
| Gelatin 75 (pork) | 4.00 |
| PEG 4000 | 5.00 |
| Total | 100.00 |

### Preparation process:

The pH of the water in which Fexofenadine HCl is to be dissolved is adjusted to about 6.5 - 7.5 using for example sodium phosphate. Then, the pH is raised to 13, using, for example NaOH. The pH of the mixture is generally maintained at 6.5-7.5. Coloring agents are added to the solution with fexofenadine (grinding step).

Degassing is carried out to remove air bubbles. Sorbitol, sweeteners, and glycerol are added with mixing at 20-40 rpm under vacuum, and heating at 60-65°C.

Gelatin is added slowly, with mixing, preferably during a period of minimum two hours. Further degassing is carried out. Then, the pharmaceutical formulation is poured at 60-65°C into blisters and sealed.

### Example 2: MAALOX oral gum (unit weight of 3447 mg)

### Formulation POP3956:

| **Ingredients** | **% wt** |
|---|---|
| Purified water | 22.54 |
| Sorbitol 70% w/v solution | 8.16 |
| Gelatin | 10.54 |
| Glycerin | 34.84 |
| API gel | 23.20 |
| Coloring agent + flavoring agent | 0.55 |
| Sweetener | 0.16 |
| Extra water* | 2.45 |

| | |
|---|---|
| * Extra water is from the 70% sorbitol solution. | |

### Formulation POP3500 (unit weight of 3400 mg):

In an alternative process, gelatin is prepared in a primary blend containing water. This blend is combined with a secondary blend that contains the other ingredients.

| **Ingredients** | **% wt** |
|---|---|
| Purified water | 25.00 |
| Sorbitol 70% w/v solution | 8.40 |
| Gelatin | 8.11 |
| Glycerol | 34.45 |
| Magnesium hydroxide | 11.76 |
| Aluminium hydroxide | 11.76 |
| Coloring agent + flavoring agent | 0.34 |
| Sweetener | 0.16 |
| Extra water* | 2.52 |

| | |
|---|---|
| * Extra water is from the 70% sorbitol solution. | |

### Preparation process:

An initial blend containing the water, glycerin and liquid sorbitol 70% is prepared. The components are mixed and warmed to 70°C. Active pharmaceutical ingredients (API) (magnesium hydroxide and aluminum hydroxide) are added with coloring agents. Then, all of the gelatin, sweeteners and flavoring agents are added. The mixture is degassed, temperature is cooled until 60°C, and the final blend is poured into newly thermoformed blister cavities. The blister is sealed and cooled at 8°C before storage.

### Example 3: Oral gum formulation containing plant extracts for treatment of chesty cough

### Formulation (unit weight of 3400 mg):

| **Ingredients** | **% wt** |
|---|---|
| Thyme extract powder | 2.76 |
| Primula extract powder | 1.38 |
| Gelatin 160 LB 8 | 10.00 |
| Sorbitol solution 70% | 15.54 |
| Glycerol | 54.25 |
| Sucralose | 0.08 |
| Polysorbat 80 | 0.20 |
| Purified Water | 10.00 |
| Purified water (premix) | 4.74 |
| Menthol flavoring agent | 0.25 |
| Honey flavoring agent | 0.30 |
| Lemon flavoring agent | 0.40 |
| Beta caroten E160a β-Carotene | 0.10 |
| Total | 100 |

### Preparation process:

A primary blend is prepared that contains water, thyme extract, primula extract mixed at room temperature. Then sucralose, TWEEN 80, flavoring agents and beta carotene are added and mixed. In a second blend glycerol, sorbitol solution, gelatin and water are added and heated to a temperature of approximately 70°C (65°C-75°C). After mixing, a vacuum step is performed and the temperature of the solution decreases for approximately 30 minutes until approximately 50°C (45°C-55°C). Then, the primary blend is combined to the second blend. The final blend is poured into blister which is sealed and cooled.

### Example 4: Oral gum formulation containing Acetaminophen

| **Ingredients** | | | | Batch (g) |
|---|---|---|---|---|
| | | | (g/100ml) | 500 |
| Micronised Acetaminophen | | | 15.25 % | 76.25 g |
| Xanthan gum | | | 0.20 % | 1.00 g |
| Sorbitol solution 70% | | | 11.00 % | 55.00 g |
| Glycerol | | | 26.00 % | 130.00 g |
| Citric acid anydrous | | | 0.01 % | 0.05 g |
| Sucralose | | | 0.43 % | 2.17 g |
| Neohesperidin | | | 0.07 % | 0.33 g |
| Sodium chloride | | | 0.60 % | 3.00 g |
| Strawberry Flavor w/o PG | | | 0.44 % | 2.20 g |
| **Gelatine 160 LB** | | | **11.00 %** | **55.00 g** |
| Purified water | | | 35.00 % | 175.00 g |
| | | | 100.00 % | 500.00 g |
| Gum weight (g) | 1g dosage 6.56 | 500mg dosage 3.279 | | |

### Preparation process:

A primary blend is prepared that contains water, xanthan gum, micronized Acetaminophen, sucralose, sweetener, sodium chloride, anhydrous citric acid and flavoring agents are added and mixed. If preservatives are added, they are included in the primary blend. In a second blend glycerol, sorbitol solution, gelatin and water are added and heated to a temperature of approximately 70°C (65°C-75°C). After mixing, a vacuum step is performed and the temperature of the solution decreases for approximately 30 minutes until approximately 50°C (45°C-55°C). Then the primary blend is combined to the second blend. The final blend is poured into blister which is sealed and cooled.

## Claims

1. A chewable oral gum pharmaceutical formulation comprising water, sorbitol, gelatin and glycerol and at least one pharmaceutically active agent **characterized in that** with respect to water:
- the ratio of gelatin is about 0.20 - 0.80:1 by weight,
- the ratio of glycerol is about 0.65 - 3.50:1 by weight,
- the ratio of sorbitol is about 0.15 - 1.45:1 by weight.

2. The chewable oral gum pharmaceutical formulation of claim 1, wherein:
- the ratio of gelatin to water is from about 0.28 - 0.70:1 by weight,
- the ratio of glycerol to water is from about 0.70 - 3.00:1 by weight,
- the ratio of sorbitol to water is from about 0.20 - 1.40:1 by weight.

3. The chewable oral gum pharmaceutical formulation of claim 1 or 2, wherein the amount of gelatin is about 4 - 12% w/w, the amount of water is about 15 - 30% w/w, the amount of sorbitol is about 6 - 30% w/w, and the amount of glycerol is from about 30 to about 60 % w/w.

4. The chewable oral gum pharmaceutical formulation of any one of claims 1 to 3, wherein the water activity (Aw) of said formulation is less than 0.75.

5. The chewable oral pharmaceutical formulation of any one of claims 1 to 4, wherein the pharmaceutically active ingredients are chosen from fexofenadine, the combination of magnesium hydroxide and aluminum hydroxide, thyme extract, primula extract or a combination of thyme extract and primula extract, Diphenhydramine, Chlorpheniramine, Loratidine, Cetirizine, Pseudoephedrine, Guaifenesin, Dextromethorphan, Naproxen, Aspirin, Acetaminophen, Ibuprofen, Fluriprofen, Ketoprofen, Drotaverine, Codeine, magnesium salts, for example, citrate, Silibinin, Ambroxol, Hyoscine Butyl Bromide, Bromhexine, Dextromethorphan, picosulphate, or pharmaceutically acceptable salts thereof, or one or more probiotic strains, vitamin A, K, D, E, C, B1, B2, B3, B5, B6, B7, B9, or B12, or multivitamin compositions, estrogen and estrogen derivatives, unsaturated fatty acids, flavonoids, phytosterol, and combinations thereof.

6. The chewable oral gum pharmaceutical formulation of claim 5, wherein the at least one pharmaceutically active ingredient is chosen from fexofenadine, the combination of magnesium hydroxide and aluminum hydroxide, thyme extract, primula extract or a combination of thyme extract and primula extract and Acetaminophen, or pharmaceutically acceptable salts thereof.

7. The chewable oral gum pharmaceutical formulation of claim 6, wherein the at least one pharmaceutically active ingredient is fexofenadine, or pharmaceutically acceptable salts thereof, and with respect to water:
- the ratio of gelatin is from about 0.3 - 0.4:1 by weight,
- the ratio of glycerol is from about 0.7 - 1.3:1 by weight,
- the ratio of sorbitol is from about 0.4:1 - 1.4:1 by weight.

8. A chewable oral gum pharmaceutical formulation of claim 6, wherein the at least one pharmaceutically active ingredient is a combination of magnesium and aluminum hydroxides and with respect to water:
- the ratio of gelatin is from about 0.2 - 0.35:1 by weight,
- the ratio of glycerol is from about 1.2 - 1.5:1 by weight,
- the ratio of sorbitol is from about 0.1:1 - 0.4:1 by weight.

9. A chewable oral gum pharmaceutical formulation of claim 6, wherein the at least one pharmaceutically active ingredient is a combination of primula and thyme plant extracts and with respect to water:
- the ratio of gelatin is from about 0.4 - 0.7:1 by weight,
- the ratio of glycerol is from about 1.3 - 2.9:1 by weight,
- the ratio of sorbitol is from about 0.3 - 0.6:1 by weight.

10. A chewable oral gum pharmaceutical formulation of claim 6, wherein the at least one pharmaceutically active ingredient is Acetaminophen, or pharmaceutically acceptable salts thereof, and with respect to water:
- the ratio of gelatin is from about 0.1:1 to about 0.4:1 by weight,
- the ratio of glycerol is from about 1.2:1 to about 1.6:1 by weight,
- the ratio of sorbitol is from about 0.15:1 to about 0.3:1 by weight.

11. A process for preparing the chewable oral gum pharmaceutical formulation of any one of claims 1 to 10 comprising the steps of:
(i) adding the glycerol,
(ii) adding the sorbitol,
(iii) adding the water,
(iv) adding the gelatin
(v) heating the mixture to a temperature of about 45°C to 75°C,
(vi) adding the at least one pharmaceutically active ingredient,
(vii) optionally, adding the sugar substitute(s) and/or the coloring agent(s) and/or the flavoring agent(s), if present,
(viii) removing air bubbles from the resulting liquid formulation,
(ix) pouring the liquid formulation into (optionally newly) thermoformed blisters using an automated filler, wherein the amount of liquid poured into the blisters is controlled by measuring the level of the liquid formulation in blister and then, if the measured liquid level in that blister differs from a predefined target liquid level, adjusting the amount of the liquid formulation poured into the following blister so that the predefined liquid level is reached.
(x) sealing the blister pack containing the formulation with a film/sheet/cover,
(xi) cooling the filled blister pack,
(xii) optionally, cutting the blister pack into units of suitable size for packaging,
wherein, preferably, the pouring, sealing and cooling steps and the blister thermoforming are carried out in one continual production line.

12. The process according to claim 11, wherein, in step (ix), the target liquid level is 1.0 mm - 2.50 mm.

13. The process according to claim 11 or 12, wherein steps (i) to (viii) are carried out in one tank.

14. The process according to claim 11 or 12, wherein the at least one pharmaceutically active ingredient to be added in step (vi) and/or the gelatin added in step (iv) is previously dissolved or suspended in a separate tank and added to the main tank from said separate tank.

15. The process according to any one of claims 11 to 14, wherein heating temperature of formulation at heating step (v) and/or pouring step (ix) is about 45°C to about 55°C.
